(19) Europäisches Patentamt
European Patent Office
Office européen des brevets

(11) Publication number: **0 492 021 A1**

(12) **EUROPEAN PATENT APPLICATION**

(21) Application number: **90403758.7**

(22) Date of filing: **24.12.90**

(51) Int. Cl.$^5$: **A61K 31/425**, A61K 31/495, A61K 31/54, A61K 31/535, A61K 31/44

(43) Date of publication of application: **01.07.92 Bulletin 92/27**

(84) Designated Contracting States: **FR**

(71) Applicant: **MERRELL DOW PHARMACEUTICALS INC.**
**2110 East Galbraith Road**
**Cincinnati Ohio 45215-6300(US)**

(72) Inventor: **Hibert, Marcel**
**21 Rue Alfred Kastler, Domaine des Grands Prés**
**F-67114 Eschau(FR)**
Inventor: **Gittos, Maurice W.**
**16 Rue André Malraux**
**F-67115 Plobsheim(FR)**

(74) Representative: **Gillard, Marie-Louise et al**
**Cabinet Beau de Loménie 55, Rue d'Amsterdam**
**F-75008 Paris(FR)**

(54) **Use of certain 1,2-benzoisothiazol-3(2H)-1,1-dioxide derivatives in the treatment of depression and mania.**

(57) This invention relates to the manufacture of certain aromatic 2-aminoalkyl-1,2-benzoisothiazol-3(2H)one-1,1-dioxide derivatives for their use as medicaments in the treatment of depression and mania.

EP 0 492 021 A1

This invention relates to the use of certain aromatic 2-aminoalkyl-1,2-benzoisothiazol-3(2H)one-1,1-dioxide derivatives for the manufacture of medicaments in the treatment of affective disorders.

More specifically, this invention relates to the use in the manufacture of a medicament for treating affective disorders utilizing a compound of the formula

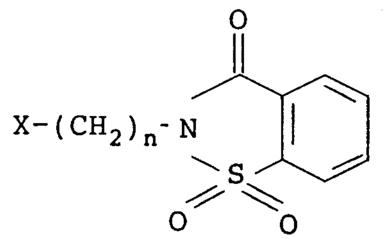

**(1)**

wherein X is selected from the group consisting of

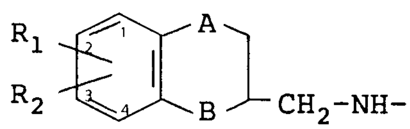

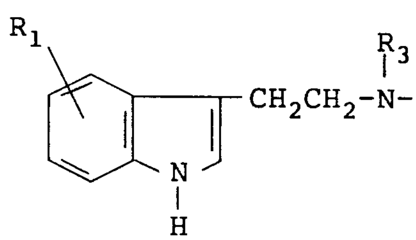

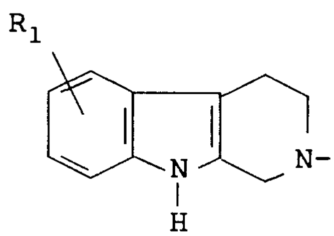

, and

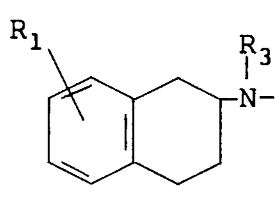

;

wherein $R_1$ is hydrogen, $C_{1-4}$ alkyl, $C_{1-4}$ alkoxy, halogeno, nitro, hydroxy, $SO_3H$, $SO_2NH_2$, or $SO_2NH_2$-$CH_2$-,

$R_2$ is hydrogen, $C_{1-4}$ alkyl, $C_{1-4}$ alkoxy, halogeno, nitro or hydroxy, and

$R_1$ and $R_2$, taken together with the carbon atoms to which they are attached, form a benzenoid moiety at the indicated 1,2- or 3,4-positions,

$R_3$ is H or $C_{1-4}$ alkyl,

n is an integer of 2 to 5 inclusive,

A and B independently are oxygen, sulfur or $NR_3$, their geometric, stereo- or optical isomers and the mixtures thereof, and the pharmaceutically acceptable acid addition salts thereof.

As used herein the term "pharmaceutically acceptable acid addition salts" is intended to apply to any non-toxic organic or inorganic acid addition salts of the base compounds represented by Formula 1. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono-, di- and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, p-hydroxybenzoic, phenylacetic, cinnamic, salicylic, 2-phenoxybenzoic acids, and sulfonic acids such as methanesulfonic acid or 2-hydroxyethanesulfonic acid. Either the mono- or the di-acid salts can be formed, and such salts can exist in either a hydrated or a substantially anhydrous form. In general, the acid addition salts of these compounds are crystalline materials which are soluble in water and in various hydrophilic organic solvents. Additionally, in comparison to their free base forms, such salts generally demonstrate higher melting points and an increased chemical stability.

As used herein, $C_{1-4}$ alkyl includes the straight and branched alkyl radicals having 1 to 4 carbon atoms such as methyl, ethyl, n-propyl, isopropyl, n-butyl, t-butyl, isobutyl and the like. $C_{1-4}$ alkoxy includes the straight and branched alkoxy radicals such as methoxy, ethoxy, n-propoxy, isopropoxy, n-butoxy, t-butoxy, isobutoxy and the like. Halogeno includes chloro, bromo and fluoro. The isomers contemplated include the position isomers, the stereoisomers and the enantiomeric isomers, including the mixtures thereof. The individual isomers of the compounds of formula I may best be prepared by processes designed to enrich the production of the desired isomers. However, mixtures may be resolved or isolated according to conventional and standard procedures well known in the art, e.g. chromatographic separation, fractional crystallization, use of optically active acids, enzymatic resolution and the like.

In essence, the compounds of formula **(1)** contain a saccharin moiety joined to the defined X moieties by a $(CH_2)_n$ alkylene bridging moiety. Preferably n represents 2 or 5. Illustrative of the X moieties are:

(2,3-dihydro-1,4-benzodioxin-2-yl)methylamino,
(2,3-dihydro-1,4-benzoxathiin-3-yl)methylamino,
(2,3-dihydro-1,4-benzodithiin-2-yl)methylamino,
(2,3-dihydro-1,4-benzoxazin-3-yl)methylamino,
(2,3-dihydro-1,4-dihydroquinazolin-2-yl)methylamino,
(2,3-dihydronaphtho[1,2-b][1,4]dioxin-2-yl)methylamino,
(2,3-dihydronaphtho[1,2-b][1,4]dioxin-3-yl)methylamino,
(indol-3-yl)ethylamino,
(1,2,3,4-tetrahydro-β-carbolinyl), and
tetralin-2-amino and the $R_1$, $R_2$ and/or $R_3$ substituted analogs.

Specific subclasses of compounds that fall within the scope of the present invention are illustrated as follows:

2-[ω-[(2,3-dihydro-1,4-benzodioxin-2-yl)methylamino]alkyl]-1,2-benzoisothiazol-3(2H)one-1,1-dioxides,

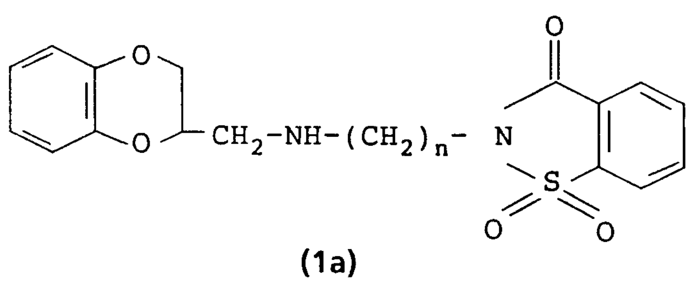

**(1a)**

2-[ω-[(2,3-dihydro-1,4-benzoxathiin-3-yl)methylamino]alkyl]-1,2-benzoisothiazol-3(2H)one-1,1-dioxides

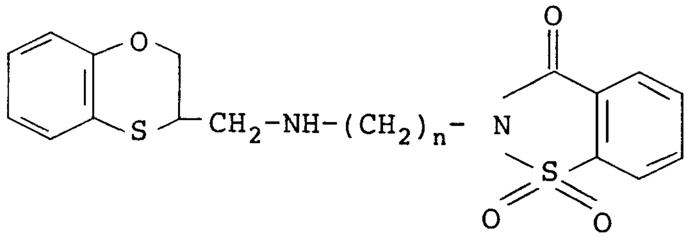

**(1b)**

2-[ω-[(2,3-dihydro-1,4-benzodithiin-2-yl)methylamino]alkyl]-1,2-benzoisothiazol-3(2H)one-1,1-dioxides

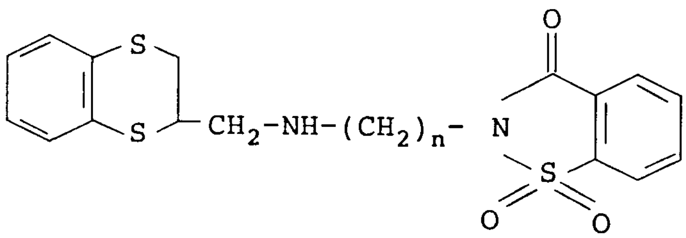

**(1c)**

2-[ω-[(2,3-dihydro-1,4-benzoxazin-3-yl)methylamino]alkyl]-1,2-benzoisothiazol-3(2H)one-1,1-dioxides

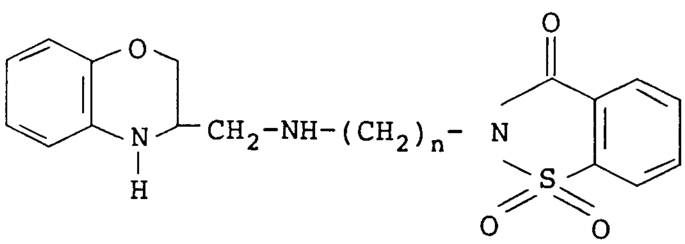

**(1d)**

2-[ω-[(2,3-dihydroquinazolin-2-yl)methylamino]alkyl]-1,2-benzoisothiazol-3(2H)one-1,1-dioxides

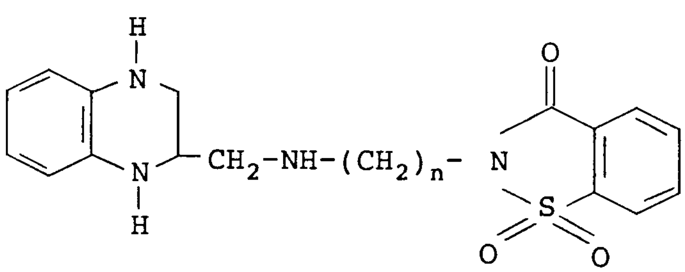

**(1e)**

2-[ω-[(2,3-dihydronaphtho[1,2-b][1,4]dioxin-2-yl)methylamino]-alkyl]-1,2-benzoisothiazol-3(2H)one-1,1-dioxides

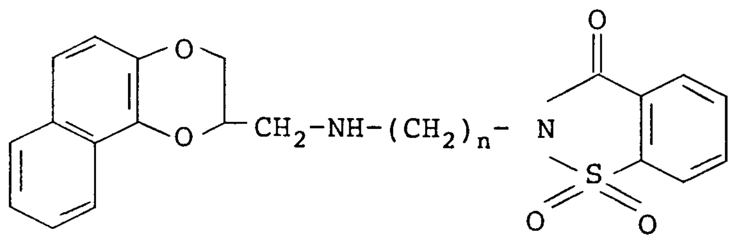

(1f)

(Compounds of this sub-class can be substituted either at the 2- or 3-position of the dioxin ring.)
2-[ω-[(indol-3-yl)ethylamino]alkyl]-1,2-benzoisothiazol-3(2H)one-1,1-dioxides

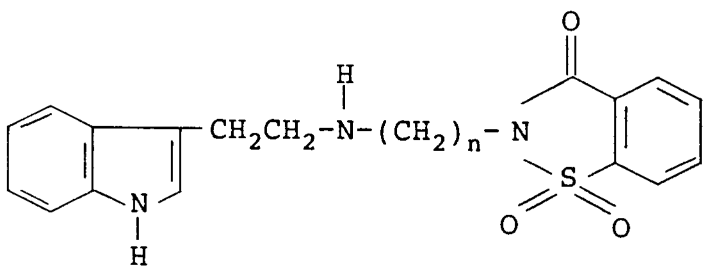

(1g)

2-[ω-[1,2,3,4-tetrahydro-β-carbolinyl]alkyl]-1,2-benzoisothiazol-3(2H)one-1,1-dioxides

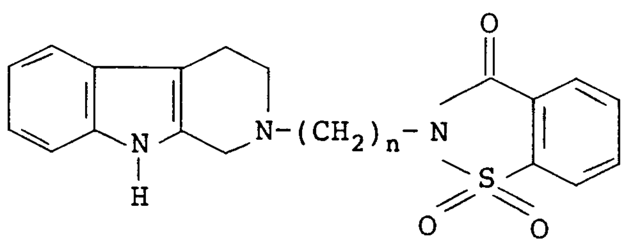

(1h)

2-[ω-[(substituted)tetralin-2-amino]alkyl]-1,2-benzoisothiazol-3(2H)one-1,1-dioxides

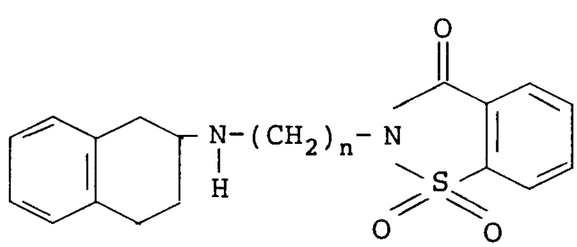

(1i)

Of course, in each of the illustrative structures **(1a)** to **(1i)**, the compounds are depicted without the $R_1$,

$R_2$ and $R_3$ substitutents (other than H), but it is understood that the illustrative structures include those compounds bearing the defined $R_1$, $R_2$ and/or $R_3$ substituents.

A preferred subclass of this invention consists of those compounds of formula **(1h)** wherein the X moiety is an $R_1$-substituted-1,2,3,4-tetrahydro-β-carboline ring system.

Another preferred subclass of this invention relates to those compounds of formula **(1f)** wherein the X-moiety is a 2,3-dihydronaphtho[1,2-][1,4]-dioxin-2-yl or 3-yl ring system.

Another preferred class of compounds of this invention relates to those compounds of formula **(1a)** wherein the X-moiety is terminally substituted by the 2,3-dihydro-1,4-benzodioxin-2-yl ring system.

The aromatic 2-aminoalkyl-1,2-benzoisothiazol-3(2H)one-1,1-dioxide derivatives of formula **(1)** can be prepared via a condensation of the appropriate nucleophilic amine of formula **(2)** with an N-alkyl-1,2-benzoisothiazol-3(2H)one-1,1-dioxide substrate of formula (3) using processes and techniques analogously known in the art, such as that shown by the following reaction scheme.

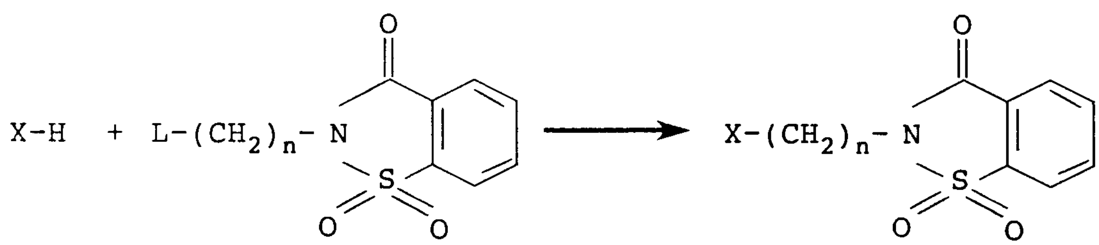

**(2)** **(3)** **(1)**

wherein X and n are as defined in formula **(1)** and the symbol (L) represents a suitable leaving group, such as chlorine, bromine, iodine, a mesylate, tosylate or hydroxyl.

Such a nucleophilic condensation is preferably conducted by reacting approximately equimolar amounts of the nucleophile **(2)** with the substrate **(3)**, for a period of from about 1 hour to 24 hours depending upon the particular reactants employed. The reaction temperature can range from about 25°C to 140°C. Preferably the reaction is conducted at a temperature ranging from 60°C to 125°C.

Further details and specific exemplifications for the nucleophilic condensation are previously described and obtainable from U.S. Patent 4,748,182 issued May 31, 1988.

The nucleophilic primary amines indicated by formula **(2)** are compounds which are either commercially available or which have been previously described in the literature. Indeed, generic teachings and the details and exemplification for the preparation of the nucleophilic primary amines of formula **(2)** may readily be obtained from U.S. Patent 4,748,182.

The compounds of formula **(3)** are essentially N-alkyl derivatives of saccharin. The leaving groups (L) for compounds of formula **(3)** can represent any group known to those skilled in the art, such as a tosylate (OTS) or a mesylate (OMS), an iodide, bromide or chloride, or hydroxyl group. Generic teachings and the details and specific exemplifications for these reactants of formula **(3)** may be obtained from U.S. Patent 4,748,182.

The following compounds are illustrative of the compounds of this invention:

2-[4-[(2,3-dihydro-1,4-benzodioxin-2-yl)methylamino]butyl]-1,2-benzoisothiazol-3(2H)one-1,1-dioxide;
2-[4-(2-[1,2,3,4]-tetrahydro-β-carbolinyl)butyl]-1,2-benzoisothiazol-3(2H)one-1,1-dioxide;
2-[4-[8-methoxytetralin-2-amino]butyl]1,2-benzoisothiazol-3(2H)one-1,1-dioxide;
2-[4-[5-methoxy-indol-3-yl)ethylamino]butyl]1,2-benzoisothiazol-3(2H)one-1,1-dioxide;
2-[4-[(2,3-dihydro-1,4-benzoxazin-3-yl)methylamino]butyl]1,2-benzoisothiazol-3(2H)one-1,1-dioxide;
2-[2-(1,2,3,4-tetrahydro-9H-pyrido[3,4-b]indol-2-yl)ethyl]-3(2H)-benzisothiazolone-1,1-dioxide, hydrochloride;
2-[3-[(2,3-dihydro[1,4]-benzodioxin-2-yl)methylamino]propyl]-3-(2H)-benzisothiazolone-1,1-dioxide;
2-[4-[(2,3-dihydronaphtho[1,2-b]-1,4-dioxin-3-yl)methylamino]-butyl]-3(2H)-benzisothiazolone-1,1-dioxide;
2-[4-[(2,3-dihydronaphtho[1,2-b]-1,4-dioxin-2-yl)methylamino]-butyl]-3(2H)-benzisothiazolone-1,1-dioxide oxalate;
2-[4-(1,2,3,4-tetrahydro-9H-pyrido[3,4-b]indol-2-yl)butyl]-3(2H)-benzisothiazolone-1,1-dioxide, hydrochloride;

U.S. Patent 4,748,182 teaches that compounds of this invention have properties which are useful for the treatment of hypertension and for treating anxiety. It recently has been found that the compounds possess properties which also will render them useful in the treatment of affective disorders, i.e., the compounds will be useful in the pharmacological treatment of depression and mania; the term affective disorders being

generic to depression and mania.

In general, the compounds of the prior art which have been found to be successful in the treatment of affective disorders have also been characterized as "mood elevators" and in recent years a plethora of the tricyclic-type of compounds have been found to be mood elevators having anti-depressant effects in endogenous and involutional depression. Illustrative of such successful tricyclic compounds are amitriphyline, desipramine, imipramine, nortriptyline, opipranol, depepin and butriptyline. Of course, many other tricyclic compounds are known but listing all such compounds is unnecessary here as they may be readily ascertained from standard works well known in the art. It is to the end-use application of these compounds to which the compounds of this invention will be useful.

Based upon standard laboratory assays as well as by comparison with compounds known to be useful in treating depression and mania, it is to be found that the compounds of this invention are effective $5HT_{1A}$ agonists capable of exerting a pharmacological effect useful for the treatment of depression and mania in the general range of 0.005 to 10 mg/kg of patient body weight, but preferably in the range of 0.05 to 5 mg/kg of patient body weight per day.

The compounds of this invention can be administered either orally, subcutaneously, intravenously, intramuscularly, intraperitoneally or rectally. The preferred route of administration is oral. The amount of compound to be administered can be any effective amount, and will vary depending upon the patient, the mode of administration and the severity of the anxiety to be treated. Repetitive daily administration of the compounds may be desirable, and will vary depending upon the patient's condition and the mode of administration.

For oral administration, an anti-depressant effective amount of a formula **(1)** compound can range from 0.005 to 10 mg/kg of patient body weight per day, preferably from 0.05 to 5 mg/kg of patient body weight per day. The preferred dose of the compounds of formula **(1)** is about 0.1 mg/kg of patient body weight per day. Pharmaceutical compositions in unit dose form can contain from 1 to 50 mg of active ingredient and can be taken one or more times per day.

For parenteral administration, an anti-depressant effective amount of a formula **(1)** compound can range from 0.005 to 10 mg/kg of patient body weight per day, preferably from 0.05 to 5 mg/kg of patient body weight per day. A parenteral composition in unit dose form can contain from 0.1 g to 10 mg of active ingredient and can be taken one or more times daily.

For oral administration the compounds can be formulated into solid or liquid preparations such as capsules, pills, tablets, lozenges, melts, powders, solutions, suspensions or emulsions. Solid dosage unit forms generally employed include capsules or tablets. Capsules can be of the ordinary gelatin type which contain additional excipients such as surfactants, lubricants and inert fillers such as lactose, sucrose and cornstarch. Additionally, the compounds of formula (1) can be tableted with conventional tablet bases such as lactose, sucrose, and cornstarch in combination with binders, such as acacia, cornstarch or gelatin, disintegrating agents such as potato starch or alginic acid, and lubricants such as stearic acid or magnesium stearate.

For parenteral administration the compounds may be administered as injectable dosages of a solution or a suspension of the compound in a physiologically acceptable diluent with or without a pharmaceutical carrier. Suitable diluents or carriers include sterile liquids such as water or oils, with or without the addition of surfactants or other pharmaceutically acceptable adjuvants. Illustrative of various oils that can be employed in the practice of this invention are those of petroleum, animal, vegetable, or synthetic origin, for example, peanut oil, soybean oil, and mineral oil. In gneral, water, saline, aqueous dextrose and related sugar solutions, ethanol and glycols such as propylene glycol or polyethylene glycol are preferred liquid carriers, particularly for injectable solutions.

Preferred compounds of this invention for the treatment of depression and mania are:

2-[4-[(2,3-dihydro-1,4-benzodioxin-2-yl)methylamino]butyl]-1,2-benzoisothiazol-3(2H)one-1,1-dioxide,

2-[2-[(2,3-dihydro-1,4-benzodioxin-2-yl)methylamino]ethyl]-1,2-benzoisothiazol-3(2H)one-1,1-dioxide, (as a salt)

2-[4-[(2,3-dihydronaphtho[1,2-b][1,4]-dioxin-3-yl)methylamino]-butyl]-1,2-benzoisothiazol-3(2H)one-1,1-dioxide,

2-[4-(1,2,3,4-tetrahydro-β-carbolinyl)butyl]-1,2-benzoisothiazol-3(2H)one-1,1-dioxide,

2-[4-[(2,3-dihydro-1,4-benzodioxin-2-yl)methylamino]butyl]-1,2-benzoisothiazol-3(2H)-one-1,1-dioxide hydrochloride,

2-[4-[(8-methoxy-2-aminotetralin )butyl]1,2-benzoisothiazol-3(2H)-one-1,1-dioxide hydrochloride.

**Claims**

**1.** The use in the manufacture of a medicament for the treatment of depression and mania of compounds of the formula

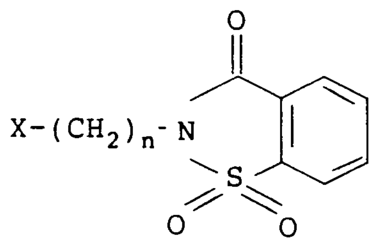

**(1)**

wherein X is selected from the group consisting of

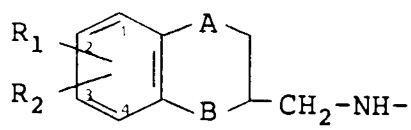

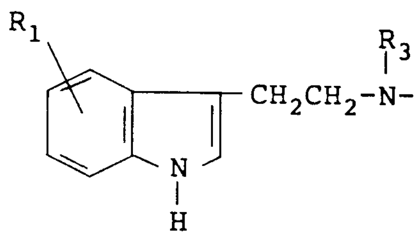

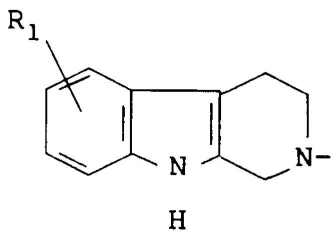

, and

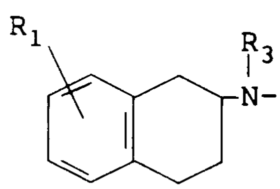

;

wherein $R_1$ is hydrogen, $C_{1-4}$ alkyl, $C_{1-4}$ alkoxy, halogeno, nitro, hydroxy, $SO_3H$, $SO_2NH_2$, or $SO_2NH_2$-$CH_2$-

$R_2$ is hydrogen, $C_{1-4}$ alkyl, $C_{1-4}$ alkoxy, halogeno, nitro or hydroxy, and

$R_1$ and $R_2$, taken together with the carbon atoms to which they are attached, form a benzenoid moiety at the indicated 1,2- or 3,4-positions,

$R_3$ is H or $C_{1-4}$ alkyl,

n is an integer of 2 to 5 inclusive,

A and B independently are oxygen, sulfur or $NR_3$, their geometric, stereo- or optical and isomers and mixtures thereof, and the pharmaceutically acceptable acid addition salts thereof.

European Patent Office

# PARTIAL EUROPEAN SEARCH REPORT

which under Rule 45 of the European Patent Convention shall be considered, for the purposes of subsequent proceedings, as the European search report

Application number
EP 90 40 3758

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-4 748 182 (MARCEL HIBERT et al.)<br>* Abstract; columns 1,2,8,12; claims 1,7,9 * | 1 | A 61 K 31/425<br>A 61 K 31/495<br>A 61 K 31/54<br>A 61 K 31/535<br>A 61 K 31/44 |
| | -- | | |
| X | DE-A-3 726 425 (BAYER AG)<br>* Pages 6,11; claims 1,10 * | 1 | |
| | -- | | |
| Y | JOURNAL OF CLINICAL PSYCHOPHARMACOLOGY, vol. 10, no. 3, suppl., 1990, pages 265-305, Williams & Wilkins;<br>M.S. EISON: "Serotonin: A common neurobiologic substrate in anxiety and depression"<br>* Page 265, abstract; pages 275-295 * | 1 | |
| | ---- | | TECHNICAL FIELDS SEARCHED (Int. Cl.4)<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:
Claims searched incompletely: 1
Claims not searched:
Reason for the limitation of the search:

See sheet -C-

| Place of search | Date of completion of the search | Examiner |
|---|---|---|
| THE HAGUE | 31-07-1991 | KRAUTBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

I. The suject matters of claim 1 are not supported by pharmacological evidence. As a result, those subject matters are speculative (EPC Articles 52(2)(a), 83 and 84). In the absence of pharmacological data, the evaluation of the technical nature of the subject matter and of the prior art are equivocal and subjective. As a consequence, it may well be that the most relevant prior art has not been cited in the search report.

II. Due to the large number of compounds, which are theoretically defined by the formula I, the search had to be restricted for economic reasons.

The search has been performed in the pertinent groups referring to the examples given in the application.